# EUROPEAN PATENT APPLICATION

(11) **EP 3 670 485 A1**
(43) Date of publication of application: **24.06.2020**
(21) Application number: 18215377.5
(22) Date of filing: 21.12.2018
(51) Int. Cl.: C07C 41/03, C07C 43/11, C07C 51/367, C07C 59/125

(54) **NOVEL ETHOXYLATES FROM RENEWABLE SOURCES**

(71) Applicant: Clariant International Ltd, 4132 Muttenz (CH)
(72) Inventor: HOEVELMANN, Felix, 84453 Mühldorf (DE); PURKAYASTHA, Nirupam, 61169 Friedberg (DE); LEINWEBER, Dirk, 65779 Kelkheim (DE)
(74) Representative: Mikulecky, Klaus

(57) **Abstract**

The present invention relates to ethoxylate surfactant compounds of general formula (I)

A-X-O-[CH₂-CH₂-O]ₙ-R¹,

wherein A is a linear C₇-C₃₃-alkyl or -alkenyl residue, wherein one of the hydrogen atoms is substituted by residue -O-[CH₂-CH₂-O]ₘ-R², X is -CH₂- or -CO-, R¹ is -H or -CH₃, R² is -H or -CH₃, and n+m = 4 to 40. Further, the invention relates to a process for synthesizing such surfactant compound, compositions comprising one or more surfactant compounds of the present invention and uses thereof.

## Description

### FIELD OF THE INVENTION

The present invention generally relates to surfactants. In particular, the present invention refers to ethoxylate surfactant compounds of general

A-X-O-[CH₂-CH₂-O]ₙ-R¹

wherein A is a linear C₇-C₃₃-alkyl or -alkenyl residue, wherein one of the hydrogen atoms is substituted by residue -O-[CH₂-CH₂-O]ₘ-R², X is -CH₂- or -CO-, R¹ is -H or -CH₃, R² is -H or -CH₃, and n+m = 4 to 40. Further, the invention deals with a process for synthesizing such surfactant compound. Moreover, the present invention refers to compositions such as solutions and formulations comprising one or more surfactant compounds of the present invention. The surfactants of the present invention can be employed in a wide range of applications. Thus, the invention also relates to uses thereof.

### BACKGROUND OF THE INVENTION

Surfactants (short for 'surface-active agents') due to their amphiphilic character, which gives them an affinity for both hydrophilic and lipophilic environments play an important role as cleaning, wetting, dispersing, emulsifying, foaming and anti-foaming agents in many practical applications and products, including: detergents, personal and home care formulations, agrochemical formulations, industrial and oil field applications, etc.

Many materials employed in the production of surfactants are traditionally derived from crude oil. Environmental, economic and sustainability questions are restricting the use of products derived from this limited resource: synthetic surfactants, for example, have been blamed for environmental incidents, particularly vis-à-vis aquatic problems in rivers and lakes. Therefore, there is a desire to identify more sustainable and biodegradeable, yet gentle and effective materials. Indeed, consumers are very interested in "natural" products including products with a high percentage of "natural" compounds and/or compounds that are derived from renewable materials. Consumers perceive compounds derived from natural materials to be gentler and more environmentally friendly. Recent industrial developments in "bio-based" chemicals are summarized, for example, in de Jong et al., "Product developments in the bio-based chemicals arena", Biofuels, Bioprod. Bioref. 6:606-624 (2012) and Foley et al., "Derivation and synthesis of renewable surfactants", Chem. Soc. Rev., 41:1499-1518 (2012).

Compounds derived from natural materials have various other benefits, including increased biodegradability and also more sustainable availability because they are not based on a limited resource. Compounds derived from plant-based resources are particularly useful since the source compound can simply be regrown. Consumers are also particularly comfortable with using compounds derived from well-known plants, especially those that are considered staple products.

Consequently, there is a need for surfactants that derived from natural materials and also have a good safety profile and low health concerns. Such new surfactants must also provide excellent performance, particularly vis-à-vis foamability.

### Unmet needs and desires

Problems regarding the surfactants in the prior art are mainly composed of materials that are derived traditionally from crude oil. As discussed before, this is undesired.

Furthermore, surfactants known in the art often contain phosphor, nitrogen and/or sulfur atoms. These heteroatoms can serve as nutrients for plants and can cause undesired stimulation of plant growth. Even algal bloom can occur. In addition, some of the chemicals used today may be toxic and/or non-biodegradable. Some of these heteroatom-containing groups are even toxic or cause undesired odors such as, e.g., mercaptanes.

Moreover, surfactants known in the art are often ionic surfactants that significantly alter the pH of a solution and may have undesired effects such as undesired effects on the skin. Often the pH is increased and aqueous compositions comprising such surfactants are rather basic.

Several surfactants based on material that is accessible from natural sources such as a plant source or a bacterial sources require rather complex and laborious synthetical routes.

In summary, there is an unmet need for surfactants which are readily synthetically obtainable from materials that are accessible from natural sources and which do not contain larger contents of nutrients or toxic agents and good performance.

### SUMMARY OF THE INVENTION

In a first aspect, the present invention relates to a surfactant compound of the following formula (I):

A-X-O-[CH₂-CH₂-O]ₙ-R¹ (I),

wherein, in formula (I):
- A: is a linear C₇-C₃₃-alkyl or -alkenyl residue, wherein one of the hydrogen atoms is substituted by a residue of formula (II):

-O-[CH₂-CH₂-O]ₘ-R² (II);
- X: is -CH₂- or -CO-;
- R¹: is -H or -CH₃;
- R²: is -H or -CH₃; and
- n+m: = 4 to 40.

In a further aspect, the present invention refers to a process for synthesizing a surfactant compound, comprising:
(i) providing one or more educt compounds of the following formula (III):

   A-X-OR¹ (III),

   wherein, in formula (III):
   - A: is a linear C₇-C₃₃-alkyl or -alkenyl residue, wherein one of the hydrogen atoms is substituted by the residue -OR², and
   - X: is -CH₂- or -CO-;
   - R¹: is -H or -CH₃, and
   - R²: is -H or -CH₃; and
(ii) reacting the one or more educt compounds with an at least 4-fold excess of ethylene oxide.

A still further aspect of the present invention relates to a surfactant compound obtainable from a method of the present invention.

In a further aspect, the present invention refers to a composition comprising at least one surfactant compound according to the present invention, wherein said composition is selected from the group consisting of a cosmetic composition, a laundry detergent composition, a rinsing agent composition, an adjuvant in crop protection, a drug product, a diagnostic product, a composition used for dehydration and desalting of oil, a composition used for enhanced oil recovery, or a composition usable in a technical process.

A still further aspect of the present invention relates to an aqueous surfactant solution comprising at least one surfactant compound according to the present invention, water, and optionally one or more further components.

In a further aspect, the present invention refers to the use of a surfactant compound or a composition according to the present invention for preparing an emulsion or for demulsifying an emulsion.

### DETAILED DESCRIPTION OF THE INVENTION

### Definitions and General

In this document, including in all embodiments of all aspects of the present invention, the following definitions apply unless specifically stated otherwise.

As far as not states otherwise, all percentages are by weight (w/w) of the total composition. "wt%" or "wt.%" means percentage by weight. All ratios are weight ratios (w/w). All ranges are inclusive and combinable. All amounts as they pertain to listed ingredients are based on the active level ('solids') and do not include carriers or byproducts that may be included in commercially available materials. The number of significant digits conveys neither a limitation on the indicated amounts nor on the accuracy of the measurements.

As far as scientifically reasonable, numerical amounts are understood to be modified by the word "about", i.e., are rounded values. For instance, 10 wt.% can be interpreted as a range of from 9.50 wt.% to 10.49 wt.%.

As far as not states otherwise, the measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein, "min" means "minute" or "minutes". Herein, "mol" means mole. Herein "g" following a number means "gram" or "grams".

Herein, "comprising" means that other steps and other ingredients can optionally be present in addition. "Comprising" encompasses the terms "consisting of" and "consisting essentially of".

The compositions, formulations, methods and uses of the present invention can comprise, consist of, and consist essentially of the elements and limitations of the invention described herein, as well as any of the additional or optional ingredients, components, steps, or limitations described herein. The terms "composition" includes solutions and formulations.

Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. Where amount ranges are given, these are to be understood as being the total amount of said ingredient in the composition, or where more than one species fall within the scope of the ingredient definition, the total amount of all ingredients fitting that definition, in the composition.

"Independently selected from," means that the referenced groups can be the same, different, or a mixture thereof, unless the context clearly indicates otherwise.

"Molecular weight" or "M.Wt." or "MW" and grammatical equivalents mean the number average molecular weight.

"Viscosity" is measured at 50°C in millipascal-seconds (mPas) using an Anton-Paar Rheometer.

"Water-soluble" refers to any material that is sufficiently soluble in water to form a clear solution to the naked eye at a concentration of 0.1% by weight of the material in water at 25°C. The term "water-insoluble" refers to any material that is not "water-soluble".

"Substantially free from" or "substantially free of" means less than 1%, or less than 0.8%, or less than 0.5%, or less than 0.3%, or about 0%, by total weight of the composition.

"Polymer" means a chemical formed from the polymerisation of two or more monomers. The term "polymer" shall include all materials made by the polymerisation of monomers as well as natural polymers. Polymers made from only one type of monomer are called homopolymers. Herein, a polymer comprises at least two monomers. Polymers made from two or more different types of monomers are called copolymers. The distribution of the different monomers can be random, alternating or block-wise (i.e. block copolymer). The term "polymer" used herein includes any type of polymer including homopolymers and copolymers. Small polymers (e.g., comprising up to 100 monomer units, in particular up to 10 monomer units) may also be designated as "oligomers". For example, an oligomer may be a dimer, trimer, tetramer, pentamer, hexamer, heptamer, octamer, nonamer or decamer.

"Monomer" means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit" means a monomer that has already been polymerised i.e. is part of a polymer.

The term "linear alkyl" is understood in the broadest sense as any linear saturated moiety that is composed of carbon and hydrogen atoms. For example, a linear alkyl can be described by the formula -(CH₂)ₓ-CH₃, wherein, x can be an integer or 0 or greater. In the context of the present invention, x is an integer as defined in the respective formula.

The term "linear alkenyl" is understood in the broadest sense as any linear unsaturated moiety, comprising one or more double bonds. In other words, a linear alkenyl is a linear alkyl, wherein one or more -CH₂-CH₂- are replaced by -CH=CH-. This also includes the possibility that -CH₂-CH₂-H (i.e., -CH₂-CH₃) may be replaced by -CH=CH-H (i.e., -CH=CH₂) when the double bond is located at the end.

"Aqueous" means that a composition or environment is mainly composed of water, i.e., that a composition or environment comprises more than 50 wt.% of water.

Surprisingly, it has now been found that effective non-ionic surfactants as described herein can be readily synthetically obtainable by ethoxylation of natural occurring diol compounds. These surfactants bear good performance, are environmentally and skin friendly and do not contain larger contents of plant nutrients.

The details of the invention and its aspects are provided hereinafter.

A first aspect of the present invention refers to a surfactant compound of the following formula (I):

A-X-O-[CH₂-CH₂-O]ₙ-R¹ (I),

wherein, in formula (I):
- A: is a linear C₇-C₃₃-alkyl or -alkenyl residue, wherein one of the hydrogen atoms is substituted by a residue of formula (II):

-O-[CH₂-CH₂-O]ₘ-R² (II);
- X: is -CH₂- or -CO-;
- R¹: is -H or -CH₃;
- R²: is -H or -CH₃; and
- n+m: = 4 to 40.

The educt compounds of the surfactant compound of the present invention can be optionally obtained from a natural source such as a plant source or a bacterial source.

Optionally, two or more units derived from formula (I), in particular when comprising one or more ester groups, may be conjugated with each other and thereby form an oligomer or polymer. It will be understood that such oligomers may also be embraced by formula (I) and the present invention.

In general, n and m can each independently from another be an integer from 0 to 40, wherein the sum of n and m (n+m) is an integer from 4 to 40 (n+m = 4 to 40). In at least one embodiment, n and m are each at least 1. In a further embodiment, n and m may be each at least 1 or at least 2. Accordingly, the integers n and m preferably are each independently from another an integer from 1 to 39. In a preferred embodiment, n+m = 5 to 25. In a preferred embodiment, n+m is selected from the group consisting of an integer of from 2.5 to 7.5, an integer of from 5 to 10, an integer of from 7.5 to 12.5, an integer of from 10 to 15, an integer of from 12.5 to 17.5, an integer of from 15 to 20, an integer of from 17.5 to 22.5, an integer of from 20 to 25, and an integer of from 22.5 to 27.5. In a preferred embodiment, n+m is selected from the group consisting of an integer of 5, 6, 7, 8, 9, 10, 11, 12, 13, 14, 15, 16, 17, 18, 19, 20, 21, 22, 23, 24, and 25. In a preferred embodiment, n+m is selected from the group consisting of an integer of 5, 10, 15, 20, and 25.

In one embodiment, R¹ is -H. In an alternative embodiment, R¹ is -CH₃. In a preferred embodiment, R² is -H. In an alternative embodiment, R² is -CH₃.

In a preferred embodiment, A is a linear C₉-C₁₉-alkyl or -alkenyl. In another preferred embodiment, A is a linear C₁₀-C₁₈-alkyl or -alkenyl, a linear C₁₁-C₁₇-alkyl or -alkenyl, or a linear C₁₂-C₁₉-alkyl or -alkenyl. In another preferred embodiment, A is selected from the group consisting of a linear C₁₀-alkyl or -alkenyl, a linear C₁₁-alkyl or -alkenyl, a linear C₁₂-alkyl or -alkenyl, a linear C₁₃-alkyl or -alkenyl, a linear C₁₄-alkyl or -alkenyl, a linear C₁₅-alkyl or -alkenyl, a linear C₁₆-alkyl or -alkenyl, a linear C₁₇-alkyl or -alkenyl, a linear C₁₈-alkyl or -alkenyl, and a linear C₁₉-alkyl or -alkenyl.

In a preferred embodiment, A is a linear C₉-C₁₉-alkyl. In another preferred embodiment, A is a linear C₁₀-C₁₈-alkyl, a linear C₁₁-C₁₇-alkyl, or a linear C₁₂-C₁₉-alkyl. In another preferred embodiment, A is selected from the group consisting of a linear C₁₀-alkyl, a linear C₁₁-alkyl, a linear C₁₂-alkyl, a linear C₁₃-alkyl, a linear C₁₄-alkyl, a linear C₁₅-alkyl, a linear C₁₆-alkyl, a linear C₁₇-alkyl, a linear C₁₈-alkyl, and a linear C₁₉-alkyl.

In another preferred embodiment, A is a linear C₉-C₁₉-alkenyl. In another preferred embodiment, A is a linear C₁₀-C₁₈-alkenyl, a linear C₁₁-C₁₇-alkenyl, or a linear C₁₂-C₁₉-alkenyl. In another preferred embodiment, A is selected from the group consisting of a linear C₁₀-alkenyl, a linear C₁₁-alkenyl, a linear C₁₂-alkenyl, a linear C₁₃-alkenyl, a linear C₁₄-alkenyl, a linear C₁₅-alkenyl, a linear C₁₆-alkenyl, a linear C₁₇-alkenyl, a linear C₁₈-alkenyl, and a linear C₁₉-alkenyl.

In a preferred embodiment, the surfactant compound of the present invention is further characterized by one or more of the following features selected from the group consisting of
- A: is a linear C₉-C₁₉-alkyl or -alkenyl, wherein one of the hydrogen atoms is substituted by a residue of formula (II);
- R²: is -H; and
- n+m: = 5 to 25.

In a preferred embodiment, the surfactant compound of the present invention is further characterized by the following features selected from the group consisting of
- A: is a linear C₉-C₁₉-alkyl or -alkenyl, wherein one of the hydrogen atoms is substituted by a residue of formula (II);
- R²: is -H; and
- n+m: = 5 to 25.

In a preferred embodiment, A is a linear C₁₁-C₁₇-alkyl or -alkenyl, wherein one of the hydrogen atoms is substituted by a residue of formula (II). In a preferred embodiment, A is a linear C₁₁-C₁₅-alkyl or -alkenyl, wherein one of the hydrogen atoms is substituted by a residue of formula (II).

In a preferred embodiment, the surfactant compound of the present invention is any one of the following formulae (Ia) or (Ib) or comprises a structure of any one of the following formulae (Ia) or (Ib):

Aₐ-CH(O-[CH₂-CH₂-O]ₘ-R²)-CH₂-X-O-[CH₂-CH₂-O]ₙ-R¹ (Ia),

or

R²-[O-CH₂-CH₂]ₘ-O-A_{b}-X-O-[CH₂-CH₂-O]ₙ-R¹ (Ib),

wherein, in formulae (Ia) and (Ib):
- Aₐ: is a linear C₅-C₃₁-alkyl or -alkenyl residue;
- A_{b}: is a linear C₇-C₃₃-alkylene or -alkenylene residue; and
the remaining residues X, R¹, R², n and m are defined as throughout the present invention,
wherein one or more -CH₂-CH₂- can optionally each be replaced by -CH=CH-.

Optionally, two or more of the structures of formula (Ia) and/or (Ib) may be conjugated with each other.

In a preferred embodiment, as far as present, Aₐ is a linear C₆-C₂₅-alkyl or -alkenyl residue, a linear C₇-C₂₀-alkyl or -alkenyl residue, a linear C₈-C₁₅-alkyl or -alkenyl residue, a linear C₉-C₁₄-alkyl or -alkenyl residue. In a preferred embodiment, as far as present, Aₐ is a linear C₆-C₂₅-alkyl residue, a linear C₇-C₂₀-alkyl residue, a linear C₈-C₁₅-alkyl residue, a linear C₉-C₁₄-alkyl residue. In another preferred embodiment, as far as present, Aₐ is a linear C₆-C₂₅-alkenyl residue, a linear C₇-C₂₀-alkenyl residue, a linear C₈-C₁₅-alkenyl residue, a linear C₉-C₁₄-alkenyl residue.

In a preferred embodiment, as far as present, A_{b} is a linear C₈-C₂₇-alkyl or -alkenyl residue, a linear C₉-2alkyl or -alkenyl residue, a linear C₁₀-C₁₇-alkyl or -alkenyl residue, a linear C₁₁-C₁₆-alkyl or -alkenyl residue. In a preferred embodiment, as far as present, Aₐ is a linear C₈-C₂₇-alkyl residue, a linear C₉-C₂₂-alkyl residue, a linear C₁₀-C₁₇-alkyl residue, a linear C₁₁-C₁₆-alkyl residue. In a preferred embodiment, as far as present, Aₐ is a linear C₈-C₂₇-alkenyl residue, a linear C₉-C₂₂-alkenyl residue, a linear C₁₀-C₁₇-alkenyl residue, a linear C₁₁-C₁₆-alkenyl residue.

In a preferred embodiment, the surfactant compound of the present invention is any one of the following formulae (Ic) or (Id) or comprises a structure of any one of the following formulae (Ic) or (Id):

H₃C-(CH₂)ₒ-CH(O-[CH₂-CH₂-O]ₘ-R²)-CH₂-X-O-[CH₂-CH₂-O]ₙ-R¹ (Ic),

or

R²-[O-CH₂-CH₂]ₘ-O-(CH₂)ₚ-X-O-[CH₂-CH₂-O]ₙ-R¹ (Id),

wherein, in formulae (Ic) and (Id):
- o: is an integer from 6 to 20;
- p: is an integer from 9 to 23; and
the remaining residues X, R¹, R², n and m are defined as throughout the present invention,
wherein one or more -CH₂-CH₂- can optionally each be replaced by -CH=CH-.

In a preferred embodiment, as far as present, o is an integer from 7 to 19, from 8 to 18, from 9 to 19, or from 10 to 18. In a preferred embodiment, as far as present, o is an integer selected from the group consisting of 6, 7, 8, 9,10, 11, 12, 13, 14, 15, 16, 17, 18, 19, and 20.

In a preferred embodiment, as far as present, p is an integer from 10 to 22, or from 11 to 20. In a preferred embodiment, as far as present, o is an integer selected from the group consisting of 9,10, 11, 12, 13, 14, 15, 16, 17, 18, 19, 20. 21. 22 and 23.

Optionally, two or more of the structures of formula (Ic) and/or (Id) may be conjugated with each other.

In a preferred embodiment, the surfactant compound of the present invention is any one of the following formulae (Ie) to (Ih) or comprises a structure of any one of the following formulae (Ie) to (Ih):

H₃C-(CH₂)ₒ-CH(O-[CH₂-CH₂-O]ₘ-R²)-(CH₂)₂-O-[CH₂-CH₂-O]ₙ-R¹ (Ie),

H₃C-(CH₂)ₒ-CH(O-[CH₂-CH₂-O]ₘ-R²)-CH₂-CO-O-[CH₂-CH₂-O]ₙ-R¹ (If),

R²-[O-CH₂-CH₂]ₘ-O-(CH₂)ₚ-CH₂-O-[CH₂-CH₂-O]ₙ-R¹ (Ig),

or

R²-[O-CH₂-CH₂]ₘ-O-(CH₂)ₚ-CO-O-[CH₂-CH₂-O]ₙ-R¹ (Ih),

wherein, in formulae (Ie) to (Ih):
- o: is an integer from 6 to 20;
- p: is an integer from 9 to 23; and
the remaining residues X, R¹, R², n and m are defined as throughout the present invention,
wherein one or more -CH₂-CH₂- can optionally each be replaced by -CH=CH-.

Optionally, two or more of the structures of formula (Ie), (If), (Ig) and/or (Ih) may be conjugated with each other.

In a preferred embodiment, the surfactant compound of the present invention is any one of the following formulae or comprises any one of the following formulae: 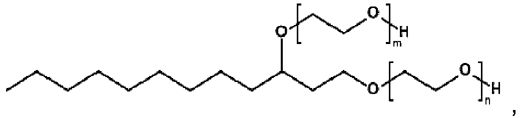 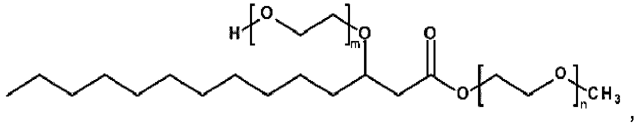 or 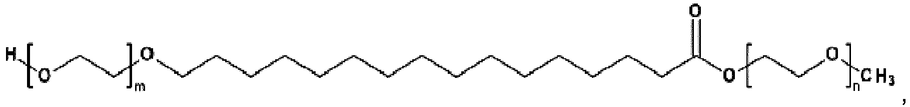 wherein n and m are defined as throughout the present invention, and wherein one or more -CH₂-CH₂- can optionally each be replaced by -CH=CH-.

Optionally, these structures may be conjugated with each other.

As indicated above, in an embodiment of the present invention, two or more of the structures of formula (I) are conjugated with each other. It will be understood that, in this case, one or more of the atoms is replaced by a bond to another moiety or a linker moiety bond to another moiety. In this context, another moiety may be a unit that is derived from formula (I). Two or more units derived from formula (I) are conjugated with each other and thereby form an oligomer or polymer. In other words, a surfactant compound of the following formula (I) or a fraction thereof may optionally also undergo an intermolecular reaction yielding in one or more higher molecular weight products. In particular, the hydroxyl-ester compounds (i.e., surfactant compounds of formula (I) comprising one or more ester groups) may optionally also undergo an intermolecular reaction yielding in one or more higher molecular weight products. It will be understood that, in addition to the surfactant compound of the following formula (I) as such, also oligomers and polymers comprising two or more of such moieties of derived from formula (I) may also be embraced by the formulae of the present invention. This is emphasized by characterization of the surfactant compound of the present invention as comprising any one of the above depicted formulae and/or further characteristics.

In a second aspect, the invention provides a process for synthesizing a surfactant compound, comprising:
(i) providing one or more educt compounds of the following formula (III):

   A-X-OR¹ (III),

   wherein, in formula (III):
   - A: is a linear C₇-C₃₃-alkyl or -alkenyl residue, wherein one of the hydrogen atoms is substituted by the residue -OR², and
   - X: is -CH₂- or -CO-;
   - R¹: is -H or -CH₃, and
   - R²: is -H or -CH₃; and
(ii) reacting the one or more educt compounds with an at least 4fold excess of ethylene oxide.

It will be understood that the definitions and preferred embodiments as laid out in the context of the surfactant compound of the present invention *mutatis mutandis* apply to the process of the present invention. In a preferred embodiment, the surfactant compound obtained from the method is as defined in the context of the surfactant compound of the present invention. Accordingly, in an educt compound of formula (III) the residues A, X, R¹ and R² may be defined as laid out in the context of formula (I) and (II) herein.

In a preferred embodiment, the educt compounds of formula (III) in obtained from a natural source such as a plant source or a bacterial source.

In a preferred embodiment, the educt compound is any of the following formulae (IIIa) or (IIIb):

Aₐ-CH(O-R²)-CH₂-X-O-R¹ (IIIa),

or

R²-O-A_{b}-X-O-R¹ (IIIb),

wherein Aₐ, A_{b}, X, R¹, and R², are defined as in the context of the surfactant compound.

In a preferred embodiment, the educt compound is any of the following formulae (IIIc) or (IIId):

H₃C-(CH₂)ₒ-CH(O-R²)-CH₂-X-O-R¹ (Ic),

or

R²-O-(CH₂)ₚ-X-O-R¹ (Id),

wherein o, p, X, R¹, and R², are defined as in the context of the surfactant compound.

In a preferred embodiment, the educt compound is any one of the following formulae (IIIe) to (IIIh):

H₃C-(CH₂)ₒ-CH(O-R²)-(CH₂)₂-O-R¹ (Ie),

H₃C-(CH₂)ₒ-CH(O-R²)-CH₂-CO-O-R¹ (If),

R²-O-(CH₂)ₚ-CH₂-O-R¹ (Ig),

or

R²-O-(CH₂)ₚ-CO-O-R¹ (Ih),

wherein o, p, X, R¹, and R², are defined as in the context of the surfactant compound.

In a preferred embodiment, the educt compound of the present invention is any one of the following formulae:

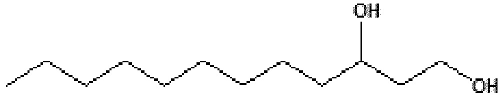

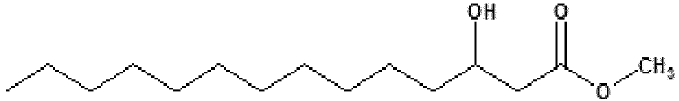

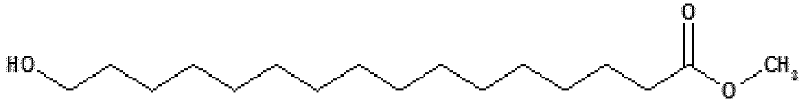

The step (ii) of reacting the components may be conducted at any conditions suitible for this purpose.

The excess of ethylene oxide is at least 4fold. As used herein, excess is stoichiometric excess of ethylene oxide over the oxygen atoms chemically accessible for ethylene oxide (i.e., oxygen atoms bound via single bonds such as those present in hydroxyl groups (-OH), ether groups (-O-), carboxyl groups (-CO-OH) and/or ester groups (-CO-O-)).

As indicated above, typically there are two of such oxygen atoms chemically accessible for ethylene oxide in an educt compound of formula (III). Accordingly, the molar content of ethylene oxide is typically at least double of the molar content of an educt compound of formula (III).

In one embodiment, ethylene oxide is used in a 4 to 50fold excess (over accessible oxygens). In another preferred embodiment, ethylene oxide is used in a 4 to 40fold excess, a 4 to 30fold excess, or in a 5 to 25fold excess. In a preferred embodiment, the excess of ethylene oxide is from 2.5fold to 7.5fold, from 5fold to 10fold, from 7.5fold to 12.5fold, from 10fold to 15fold, from 12.5fold to 17.5fold, from 15fold to 20fold, from 17.5fold to 22.5fold, from 20fold to 25fold, or from 22.5fold to 27.5fold. In a preferred embodiment, the excess of ethylene oxide is selected from the group consisting of an integer of 5fold, 6 fold, 7 fold, 8 fold, 9 fold, 10 fold, 11 fold, 12 fold, 13 fold, 14 fold, 15 fold, 16 fold, 17 fold, 18 fold, 19 fold, 20 fold, 21 fold, 22 fold, 23 fold, 24 fold, and 25 fold. In a preferred embodiment, the excess of ethylene oxide is selected from the group consisting of an integer of 5 fold, 10 fold, 15 fold, 20 fold, and 25 fold.

In a preferred embodiment, step (ii) is conducted at basic conditions, i.e., at a pH >7. In a preferred embodiment, step (ii) is conducted in a potassium hydroxide (KOH) solution, in particular a 20-50 wt.% KOH solution. KOH may serve as a catalyst.

In a preceding step, before adding ethylene oxide to the compound of formula (III), water can be removed. Optionally, water removal may be achieved by applying a (partial) vacuum and/or an elevated temperature >60°C, in particular 95°C, for example around 100°C. In a preferred embodiment, a protection gas such as, e.g., nitrogen, may be used. Ethylene oxide may be added at an elevated pressure such as, e.g, 1.1-5 bar, in particular 2-4 bar, for instance around 3 bar. In a preferred embodiment, reaction of the compound of formula (III) and ethylene oxide is conducted at an elevated temperature. In a preferred embodiment, reaction of the compound of formula (III) and ethylene oxide is conducted at a temperature in the range of 60 to 200°C, 80 to 180°C, 100 to 170°C, or 145-165°C. After the desired amount of ethylene oxide is added, the reaction may be optionally conducted further at an essentially constant pressure to finalize reaction and to increase yields further.

In a subsequent step, the reaction may be cooled. Hydroxyl values and/or saponification values may be determined, for instance, by means of titration to qualify the reaction with the required amount of ethylene oxide.

In a third aspect, the invention relates to a surfactant compound obtainable from a method of the present invention.

It will be understood that the definitions and preferred embodiments as laid out in the context of the surfactant compound and the method of the present invention *mutatis mutandis* apply to the surfactant compound obtainable from such method.

In a fourth aspect, the invention refers to a composition comprising at least one surfactant compound according to the present invention, wherein said composition is selected from the group consisting of a cosmetic composition, a laundry detergent composition, a rinsing agent composition, an adjuvant in crop protection, a drug product, a diagnostic product, a composition used for dehydration and desalting of oil, a composition used for enhanced oil recovery, or a composition usable in a technical process.

It will be understood that the definitions and preferred embodiments as laid out in the context of the surfactant compounds and the method of the present invention *mutatis mutandis* apply to the composition comprising at least one surfactant compound.

Such composition comprising at least one surfactant compound may be any type of composition. For instance such composition may be an a solution (e.g. an aqueous solution, an alcoholic solution, a solution in one or more other organic solvents or a solution is a mixture of different solvents), an emulsion (e.g., an water-in-oil (W/O) emulsion, an oil-in-water (O/W) emulsion, an oil-in-water-in-oil (O/W/O) emulsion, or an water-in-oil-in-water (W/O/W) emulsion), a solid (e.g., a powder). The composition may be a liquid formulation, a viscous formulation (e.g., a gel, or a cream), a solid formulation (e.g. a powder, a waxy solid or a pasty good).

Such composition comprising at least one surfactant compound may be used for personal care (e.g., in or as a cosmetic composition (e.g., a shower gel, a cleansing composition, a hair dye, a toothpaste, a shampoo, a body lotion, a facial or body cream, etc., in a consumer good (e.g., in or as a laundry detergent composition, a rinsing agent composition or a cleaning agent), for technical purposes (e.g., in or as an adjuvant in crop protection, a composition used for dehydration and desalting of oil, a composition used for enhanced oil recovery or a composition usable in another technical process) and/or for medicinal purposes (e.g., in or as a drug product or a diagnostic product).

In a preferred embodiment, the composition comprises at least two compounds of formula (I), wherein the first compound bears a saturated alkyl residue as residue A (or Aₐ or A_{b} respectively) and the other compound bears one double bond in the residue A (or Aₐ or A_{b} respectively). In a preferred embodiment, the ratio of compounds bearing a saturated alkyl residue as residue A (or Aₐ or A_{b} respectively) and those bearing one double bond in the residue A (or Aₐ or A_{b} respectively) is between 2:1 and 1:2, or is approximately 1:1 (i.e., between 1.49:1.00 and 1.00:1.49).

The composition may optionally comprise one or more further components that are suitible for the intended purpose. For instance, such further component may be selected from the group consisting of a dye, a chelator, an aroma component, a perfume component, a buffer agent, an acidity regulator, a fatty acid, a further surfactant, a humectant, a thickener, a polymer beads, an organic or inorganic salt, insoluble organic or inorganic particulates, a drug or diagnostic agent, a galenic agent, a filler, an enzyme, a polypeptide, a sugar, an educt compound of formula (III), and a compound having two or more of the aforementioned functionalities.

In a preferred embodiment, the composition comprises 0.01 to 90 wt.% of the surfactant compound of formula (I). In a preferred embodiment, the composition comprises 0.1 to 50 wt.%, 0.2 to 20 wt.% or 0.3 to 10 wt.%, or 0.5 to 5 wt.% of the surfactant compound of formula (I).

In a fifth aspect, the invention relates to an aqueous surfactant solution comprising at least one surfactant compound according to the present invention, water, and optionally one or more further components.

It will be understood that the definitions and preferred embodiments as laid out in the context of the surfactant compounds and the method of the present invention and a composition comprising at least one surfactant compound of the present invention *mutatis mutandis* apply to the aqueous surfactant solution.

The one or more further components may be any compounds suitible for the intended purpose. For instance, such further component may be selected from the group consisting of a dye, a chelator, an aroma component, a perfume component, a buffer agent, an acidity regulator, a fatty acid, a further surfactant, a humectant, a thickener, a polymer beads, an organic or inorganic salt, insoluble organic or inorganic particulates, a filler, an enzyme, a drug or diagnostic agent, a galenic agent, a polypeptide, a sugar, an educt compound of formula (III), and a compound having two or more of the aforementioned functionalities.

In a preferred embodiment, the aqueous surfactant solution comprises at least two compounds of formula (I), wherein the first compound bears a saturated alkyl residue as residue A (or Aₐ or A_{b} respectively) and the other compound bears one double bond in the residue A (or Aₐ or A_{b} respectively). In a preferred embodiment, the ratio of compounds bearing a saturated alkyl residue as residue A (or Aₐ or A_{b} respectively) and those bearing one double bond in the residue A (or Aₐ or A_{b} respectively) is between 2:1 and 1:2, or is approximately 1:1 (i.e., between 1.49:1.00 and 1.00:1.49).

In a preferred embodiment, the aqueous surfactant solution comprises 0.01 to 50 wt.% of the surfactant compound of formula (I). In a preferred embodiment, the aqueous surfactant solution comprises 0.1 to 20 wt.% or 0.2 to 10 wt.% or 0.5 to 5 wt.% of the surfactant compound of formula (I).

In a preferred embodiment, the aqueous surfactant solution comprises:
0.1 to 10 wt.% of the surfactant compound of formula (I);
50 to 99.9 wt.% or water; and optionally
0 to 49.9 wt.% of one or more further ingredients.

In a preferred embodiment, the aqueous surfactant solution comprises:
0.1 to 5 wt.% of the surfactant compound of formula (I);
74.9 to 99.9 wt.% or water; and optionally
0 to 20 wt.% of one or more further ingredients.

In a sixth aspect, the invention relates to the use of a surfactant compound or a composition according to the present invention for preparing an emulsion or for demulsifying an emulsion.

Accordingly, one embodiment refers to the use of a surfactant compound or a composition according to the present invention for preparing an emulsion. Another embodiment refers to the use of a surfactant compound or a composition according to the present invention for demulsifying an emulsion.

In a preferred embodiment, the use is for preparing an emulsion or for demulsifying an emulsion in an aqueous environment such as, e.g., an aqueous solution, an aqueous cream or gel, or an aqueous an emulsion (e.g., an water-in-oil (W/O) emulsion, an oil-in-water (O/W) emulsion, an oil-in-water-in-oil (O/W/O) emulsion, or an water-in-oil-in-water (W/O/W) emulsion).

In a preferred embodiment, the use comprises the step of admixing a composition, in particular an aqueous environment, with a surfactant compound or a composition according to the present invention.

In other words, this aspect of the invention relates to a method for preparing an emulsion or for demulsifying an emulsion, said method comprising the step of admixing a composition, in particular an aqueous environment, with a surfactant compound or a composition according to the present invention.

It will be understood that the definitions and preferred embodiments as laid out in the context of the surfactant compounds and the method of the present invention and a composition comprising at least one surfactant compound and the aqueous surfactant solution of the present invention *mutatis mutandis* apply to the use thereof.

Depending on the intended purpose, the concentration ranges of the surfactant compound will be adjusted.

### EXAMPLES

The following examples and claims further illustrate various properties of the present invention.

The preferred embodiments as described below will be applicable to all aspects of this invention.

### Materials

The following raw materials were obtained from REG (Renewable Energy Group, Inc., Ames, Iowa, USA).
Dodecane-1,3-diol, partially unsaturated, containing mainly C₁₂ saturated and mono unsaturated-1,3-diol
Methyl-3-hydroxytetradecanoate, partially unsaturated, containing mainly C₁₄ saturated and mono unsaturated beta-hydroxy methyl ester
Methyl-16-hydroxyhexadecanoate, partially unsaturated, containing mainly C₁₆ saturated and mono unsaturated omega-hydroxy methyl ester

### General procedure for ethoxylation

Reactions were carried out according to standard alkoxylation procedure. In brief, a 1 L glass autoclave was charged with the required amount of starting material and aqueous potassium hydroxide solution as a catalyst was added. The mixture was stirred and heated to 100°C while applying vacuum for 2 h to remove water. Vacuum was compensated by nitrogen and the temperature was increased to 155°C. Ethylene oxide was added at a maximum pressure of 3 bars and the temperature was maintained at 145-165°C throughout the reaction. After the desired amount of ethylene oxide was added, the reaction was continued to reach constant pressure, prior to cooling to 70°C and filling of the product. Hydroxyl values or saponification values were determined by titration to qualify the reaction with the required amount of ethylene oxide. In a typical experiment, 185.6 g (0.88 mol, 32.5 wt%) of dodecane-1,3-diol, 0.61 (0.11 wt%) and KOH (40 wt%, aqueous solution) were reacted with 385.5 g (8.8 mol, 67.5 wt%) of ethylene oxide.

For Examples 1a-1d, the synthesis may be depicted as follows:

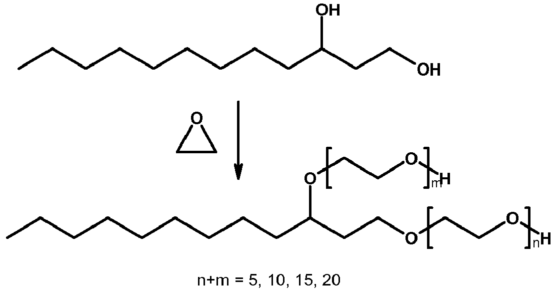

For Examples 2a-2d, the synthesis may be depicted as follows:

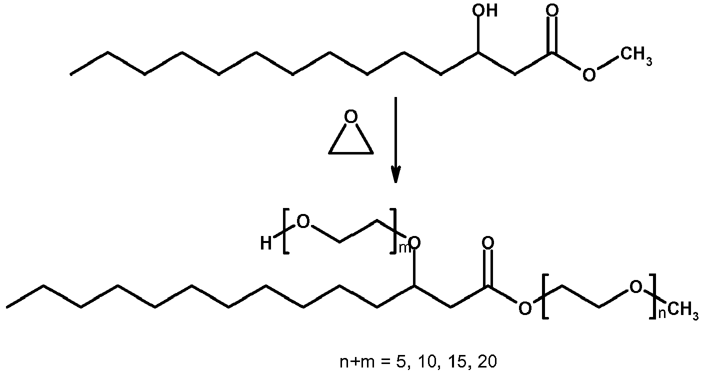

For Examples 3a-3d, the synthesis may be depicted as follows:

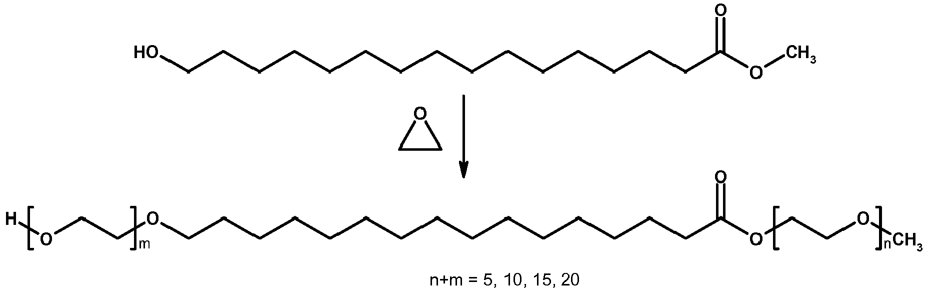

It was found that a fraction of a surfactant compound of the following formula (I) (in particular the lower two compounds (i.e. the hydroxyl-ester-compounds)) as exemplified above may optionally also undergo an intermolecular reaction yielding in one or more higher molecular weight products. It will be understood that, in addition to the surfactant compound of the following formula (I) as such, also dimers, trimers and polymers comprising two or more of such moieties of derived from formula (I) are also embraced by the present invention.

**Table 1: Chemical Components Used in Synthesis**

| example | starting material | wt.% KOH (40% aq.) | wt.% ethylene oxide | eq. EO per eq. starting material | Chemical index |
|---|---|---|---|---|---|
| 1a | dodecane-1,3-diol, partially unsaturated | 0.16 | 50.9 | 5 | 262 ^{a} |
| 1b | dodecane-1,3-diol, partially unsaturated | 0.11 | 67.5 | 10 | 173 ^{a} |
| 1c | dodecane-1,3-diol, partially unsaturated | 0.08 | 75.7 | 15 | 137 ^{a} |
| 1d | dodecane-1,3-diol, partially unsaturated | 0.06 | 80.6 | 20 | 106 ^{a} |
| 2a | Methyl-3-hydroxytetradecanoate, partially unsaturated | 0.14 | 45.4 | 5 | 151 ^{a} |
| 2b | Methyl-3-hydroxytetradecanoate, partially unsaturated | 0.10 | 62.5 | 10 | 116 ^{a} |
| 2c | Methyl-3-hydroxytetradecanoate, partially unsaturated | 0.08 | 71.4 | 15 | 89 ^{a} |
| 2d | Methyl-3-hydroxytetradecanoate, partially unsaturated | 0.06 | 76.9 | 20 | 74 ^{a} |
| 3a | Methyl-16-hydroxyhexadecanoate | 0.14 | 42.6 | 5 | 102 ^{b} |
| 3b | Methyl-16-hydroxyhexadecanoate, partially unsaturated | 0.10 | 59.7 | 10 | 80 ^{b} |
| 3c | Methyl-16-hydroxyhexadecanoate, partially unsaturated | 0.07 | 69.0 | 15 | 63 ^{b} |
| 3d | Methyl-16-hydroxyhexadecanoate, partially unsaturated | 0.06 | 74.8 | 20 | 53 ^{b} |

| | | | | | |
|---|---|---|---|---|---|
| a) Hydroxyl value by titration (mgKOH/g); b) Saponification value by titration (mgKOH/g) | | | | | |

All compounds could be synthesized in a smooth reaction without burden in a high yield.

**Table 2: Synthetic Results with Dodecane-1,3-diol**

| Example | | 1a | 1b | 1c | 1d |
|---|---|---|---|---|---|
| NMR | EO/OH | 2.5 | 4.9 | 7.2 | 9.9 |
| | EO/Alkyl | 4.8 | 10 | 15 | 21 |
| | CH₂OH | 1.3 | 1.5 | 1.7 | 1.7 |
| | CHOH | 0.61 | 0.51 | 0.42 | 0.33 |
| | Double bond | 0.38 | 0.38 | 0.37 | 0.37 |
| | CH₃ | 1 | 1 | 1 | 1 |
| viscosity | h (50°C) / mPas | 26.9 | 38.6 | 50.7 | 67.9 |

**Table 3: Synthetic Results with Methyl-3-hydroxy-tetradecanoate**

| Example | | 2a | 2b | 2c | 2d |
|---|---|---|---|---|---|
| NMR | EO/OH | 3.8 | 7.2 | 11 | 14 |
| | EO/Alkyl | 4.3 | 9.6 | 17 | 23 |
| | CH₂OH | 1.1 | 1.3 | 1.5 | 1.6 |
| | CHOH | - | - | - | - |
| | CH₂Ester | 0.72 | 0.7 | 0.73 | 0.77 |
| | DB | 0.59 | 0.53 | 0.56 | 1.1 |
| | O-CH₃ | 0.21 | 0.25 | 0.25 | 0.24 |
| | CH₃ | 1 | 1 | 1 | 1 |
| key parameters | OHZ | 151 | 116 | 89 | 74 |
| | Mw (OHZ) | 371.5 | 483.6 | 630.3 | 758.1 |
| | VZ | 96 | 61 | 44 | 32 |
| | Mw (VZ) | 584.4 | 919.7 | 1275.0 | 1753.1 |
| | Iodine value | 36 | 21 | 15 | 11 |
| | alkaline value | 1.1 | 0.7 | 0.5 | 0.4 |

**Table 4: Synthetic Results with Methyl-16-hydroxyhexadecanoate**

| Example | | 3a | 3b | 3c | 3d |
|---|---|---|---|---|---|
| NMR | EO/OH | 11 | 18 | 23 | 25 |
| | EO/(Ester+OH) | 3.8 | 6.4 | 8.9 | 11 |
| | CH₂OH (16-OH) | 0.05 | 0.05 | 0.04 | 0.04 |
| | CH₂OH (EO) | 0.31 | 0.43 | 0.48 | 0.62 |
| | CH₂Ester (EO) | 0.44 | 0.63 | 0.69 | 0.78 |
| | CH₂Ester (Alkyl) | 0.25 | 0.2 | 0.15 | 0.12 |
| | DB | 0.5 | 0.6 | 0.5 | 0.6 |
| | CH₂-C(O)- | 1 | 1 | 1 | 1 |
| | O-CH₃ | 0.3 | 0.4 | 0.41 | 0.44 |
| | Alkyl | 0.23 | 0.24 | 0.23 | 0.24 |

### Foam (collapse) test

20 mL of 1 wt.% solution of each substance were place into a graduated 100 mL measurement cylinder, which was sealed with a stopper. Foam was generated by 20 shakes. The foam height was determined as mL foam above the aqueous layer directly after shaking, after 2, 3 and 5 min.

**Table 5: Foam Height (mL)**

| substance of Example | Initial | 2 min | 3 min | 5 min |
|---|---|---|---|---|
| 1a | 86 | 62 | 34 | 15 |
| 1b | 75 | 15 | 9 | 4 |
| 1c | 97 | 26 | 13 | 10 |
| 1d | 85 | 26 | 20 | 18 |
| 2a | 4 | 3 | 3 | 3 |
| 2b | 26 | 13 | 12 | 11 |
| 2c | 33 | 17 | 16 | 14 |
| 2d | 26 | 18 | 18 | 15 |
| 3a | 26 | 14 | 13 | 12 |
| 3b | 30 | 14 | 14 | 11 |
| 3c | 32 | 14 | 13 | 8 |
| 3d | 29 | 15 | 14 | 9 |

### Dynamic surface tension

Dynamic surface tension was determined by a Kruess BP 50 bubble pressure tensiometer at a concentration of 0.3, 1.0 or 3.0 g/L of sample in water and a surface age of 200 and 400 ms at room temperature (21-23°C).

**Table 6: Dynamic Surface Tension**

| substance of Example | dynamic surface tension (mN/m) | | | |
|---|---|---|---|---|
| | 0.3 g/L | | 3 g/L | |
| | 200 ms | 400 ms | 200 ms | 400 ms |
| 1a | 46.8 | 43.3 | 35.9 | 33.7 |
| 1b | 56.6 | 54.3 | 39.5 | 38.4 |
| 1c | 54.9 | 53.4 | 43.8 | 42.5 |
| 1d | 55.0 | 53.9 | 46.8 | 45.6 |
| 2a | 70.0 | 69.4 | 59.9 | 57.2 |
| 2b | 65.6 | 63.5 | 59.6 | 57.3 |
| 2c | 64.5 | 62.9 | 57.4 | 55.2 |
| 2d | 63.7 | 61.7 | 57.0 | 54.9 |
| 3a | 60.8 | 57.5 | 54.2 | 51.6 |
| 3b | 61.9 | 58.5 | 51.8 | 49.6 |
| 3c | 58.7 | 56.0 | 51.2 | 49.3 |
| 3d | 59.3 | 56.5 | 51.6 | 49.6 |

### Wetting test

Cotton swatches (according to ISO 8022 (in the up-to-date version 2018), obtained from wfk-Testgewebe GmbH) were placed in 600 mL beaker, filled with the corresponding surfactant solution. The time between placing the swatches into the solution and sinking to the bottom was measured.

**Table 7: Wetting time (s)**

| substance of Example | content of surfactant in water | |
|---|---|---|
| | 0.5 wt.% | 0.1 wt.% |
| 1a | 1 | 19 |
| 1b | 44 | 166 |
| 1c | 170 | >300 |
| 1d | >300 | >300 |
| 2a | 63 | 272 |
| 2b | 293 | >300 |
| 2c | >300 | >300 |
| 2d | >300 | >300 |
| 3a | 143 | >300 |
| 3b | 261 | >300 |
| 3c | >300 | >300 |
| 3d | >300 | >300 |

The experimental data demonstrate that the surfactant compounds of the present invention are effective surfactants. The surfactant compounds provide excellent performance. Foamability is excellent. Foam that is stable for several minutes is formed. Further, the surface tension is significantly decreased.

## Claims

1. A surfactant compound of the following formula (I):
A-X-O-[CH₂-CH₂-O]ₙ-R¹ (I), wherein, in formula (I):
A is a linear C₇-C₃₃-alkyl or -alkenyl residue, wherein one of the hydrogen atoms is substituted by a residue of formula (II):
-O-[CH₂-CH₂-O]ₘ-R² (II);
X is -CH₂- or -CO-;
R¹ is -H or -CH₃;
R² is -H or -CH₃; and
n+m = 4 to 40.

2. The surfactant compound of claim 1, wherein n and m are each at least 1.

3. The surfactant compound of any one of claims 1 or 2, wherein said surfactant compound is further **characterized by** one or more of the following features selected from the group consisting of
A is a linear C₉-C₁₉-alkyl or -alkenyl, wherein one of the hydrogen atoms is substituted by a residue of formula (II);
R² is -H; and
n+m = 5 to 25.

4. The surfactant compound of any one of claims 1 to 3, wherein A is a linear C₁₁-C₁₇-alkyl or -alkenyl, wherein one of the hydrogen atoms is substituted by a residue of formula (II).

5. The surfactant compound of any one of claims 1 to 4, wherein said surfactant compound is any one of the following formulae (Ia) or (Ib) or comprises a structure of any one of the following formulae (Ia) or (Ib):
Aₐ-CH(O-[CH₂-CH₂-O]ₘ-R²)-CH₂-X-O-[CH₂-CH₂-O]ₙ-R¹ (Ia),
or
R²-[O-CH₂-CH₂]ₘ-O-A_{b}-X-O-[CH₂-CH₂-O]ₙ-R¹ (Ib),
wherein, in formulae (Ia) and (Ib):
Aₐ is a linear C₅-C₃₁-alkyl or -alkenyl residue;
A_{b} is a linear C₇-C₃₃-alkylene or -alkenylene residue; and
the remaining residues X, R¹, R², n and m are defined as in any one of claims 1 to 3, wherein one or more -CH₂-CH₂- can optionally each be replaced by -CH=CH-.

6. The surfactant compound of any one of claims 1 to 5, wherein said surfactant compound is any one of the following formulae (Ic) or (Id) or comprises a structure of any one of the following formulae (Ic) or (Id):
H₃C-(CH₂)ₒ-CH(O-[CH₂-CH₂-O]ₘ-R²)-CH₂-X-O-[CH₂-CH₂-O]ₙ-R¹ (Ic),
or
R²-[O-CH₂-CH₂]ₘ-O-(CH₂)ₚ-X-O-[CH₂-CH₂-O]ₙ-R¹ (Id),
wherein, in formulae (Ic) and (Id):
o is an integer from 6 to 20;
p is an integer from 9 to 23; and
the remaining residues X, R¹, R², n and m are defined as in any one of claims 1 to 3, wherein one or more -CH₂-CH₂- can optionally each be replaced by -CH=CH-.

7. The surfactant compound of any one of claims 1 to 6, wherein said surfactant compound is any one of the following formulae (le) to (Ih) or comprises a structure of any one of the following formulae (le) to (Ih):
H₃C-(CH₂)ₒ-CH(O-[CH₂-CH₂-O]ₘ-R²)-(CH₂)₂-O-[CH₂-CH₂-O]ₙ-R¹ (Ie),
H₃C-(CH₂)ₒ-CH(O-[CH₂-CH₂-O]ₘ-R²)-CH₂-CO-O-[CH₂-CH₂-O]ₙ-R¹ (If),
R²-[O-CH₂-CH₂]ₘ-O-(CH₂)ₚ-CH₂-O-[CH₂-CH₂-O]ₙ-R¹ (Ig),
or
R²-[O-CH₂-CH₂]ₘ-O-(CH₂)ₚ-CO-O-[CH₂-CH₂-O]ₙ-R¹ (Ih),
wherein, in formulae (le) to (Ih):
o is an integer from 6 to 20;
p is an integer from 9 to 23; and
the remaining residues X, R¹, R², n and m are defined as in any one of claims 1 to 3, wherein one or more -CH₂-CH₂- can optionally each be replaced by -CH=CH-.

8. The surfactant compound of any one of claims 1 to 7, wherein said surfactant compound is any one of the following formulae or comprises any one of the following formulae: 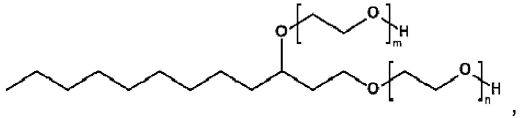 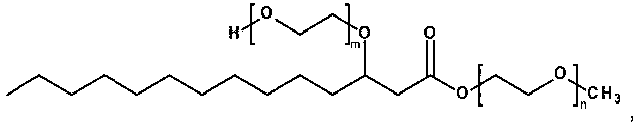 or 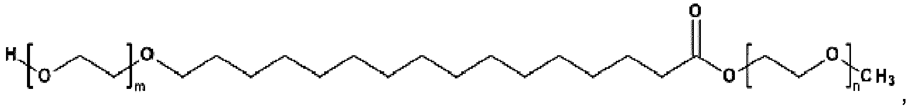 wherein n and m are defined as in any one of claims 1 to 3, and
wherein one or more -CH₂-CH₂- can optionally each be replaced by -CH=CH-.

9. The surfactant compound of any one of claims 1 to 8, wherein two or more of the structures of formula (I) are conjugated with each other.

10. A process for synthesizing a surfactant compound, comprising:
(i) providing one or more educt compounds of the following formula (III):
A-X-OR¹ (III),
wherein, in formula (III):
A is a linear C₇-C₃₃-alkyl or -alkenyl residue, wherein one of the hydrogen atoms is substituted by the residue -OR², and
X is -CH₂- or -CO-;
R¹ is -H or -CH₃, and
R² is -H or -CH₃; and
(ii) reacting the one or more educt compounds with an at least 4fold excess of ethylene oxide.

11. The method of claim 10, wherein the surfactant compound is as defined as in any one of claims 1 to 9.

12. A surfactant compound obtainable from a method of any one of claims 10 or 11.

13. A composition comprising at least one surfactant compound according to any one of claims 1 to 9 or 12, wherein said composition is selected from the group consisting of a cosmetic composition, a laundry detergent composition, a rinsing agent composition, an adjuvant in crop protection, a drug product, a diagnostic product, a composition used for dehydration and desalting of oil, a composition used for enhanced oil recovery, or a composition usable in a technical process.

14. An aqueous surfactant solution comprising at least one surfactant compound according to any one of claims 1 to 9 or 12, water, and optionally one or more further components.

15. Use of a surfactant compound according to any one of claims 1 to 9 or 12 or a composition according to claim 13 for preparing an emulsion or for demulsifying an emulsion.
